# EUROPEAN PATENT APPLICATION

(11) **EP 3 944 811 A1**
(43) Date of publication of application: **02.02.2022**
(21) Application number: 21150962.5
(22) Date of filing: 11.01.2021
(51) Int. Cl.: A61B 5/11, A61B 5/16, A61B 5/00

(54) **DEVICE AND METHOD FOR THE PREVENTION AND TREATMENT OF DYSLEXIA**

(30) Priority: 27.07.2020 IT 202000018151
(71) Applicant: Beon Solutions S.r.l., 31059 Zero Branco (TV) (IT)
(72) Inventor: Florian, Alessandro, 31059 Zero Branco (IT)
(74) Representative: Leganza, Alessandro

(57) **Abstract**

The present invention relates to the use of a device to treat dyslexia, both for the prevention phase and for the treatment of dyslexia; it also concerns a method either for the prevention of dyslexia or to improve reading skills and, finally, a diagnostic method for identifying patients potentially suitable for developing dyslexia.

## Description

### Technical Field

The present invention relates to the use of a device to treat dyslexia, both for the prevention phase and for the treatment of dyslexia; it also concerns a method either for the prevention of dyslexia or to improve reading skills and, finally, a diagnostic method for identifying patients potentially suitable for developing dyslexia.

### State of the art

Dyslexia, also known as reading disorder, is characterized by trouble with reading despite normal intelligence. Different people are affected to varying degrees. Problems may include difficulties in spelling words, reading quickly, writing words, "sounding out" words in the head, pronouncing words when reading aloud and understanding what one reads. Often these difficulties are first noticed at school. The difficulties are involuntary, and people with this disorder have a normal desire to learn. People with dyslexia have higher rates of attention deficit hyperactivity disorder (ADHD), developmental language disorders, and difficulties with numbers.

Dyslexia is believed to be caused by the interplay of genetic and environmental factors. Dyslexia that develops due to a traumatic brain injury, stroke, or dementia is called "acquired dyslexia". The underlying mechanisms of dyslexia are problems within the brain's language processing. Dyslexia is diagnosed through a series of tests of memory, vision, spelling and reading skills.

Treatment of dyslexia involves adjusting teaching methods to meet the person's needs, while not curing the underlying problem, however it may decrease the degree or impact of symptoms. Dyslexia is the most common learning disability and occurs in all areas of the world. It affects 3-7% of the population, however, up to 20% of the general population may have some degree of symptoms. While dyslexia is more often diagnosed in men, it has been suggested that it affects men and women equally. Some believe that dyslexia should be considered as a different way of learning, with both benefits and downsides.

Vision plays a key role in the knowledge of our environment during each moment of our day life. It is essential for the coordination of all our movements in natural space, for the precision to perform a task, to use a tool, for reading or writing and it is also fundamental in relationships and social communication with other humans. It is known that the stabilization of an image on the retina depends on the activity of the vestibular and visual systems. The activity of each of these two systems depends on the oscillation frequency of the head. For instance, at low frequencies (<0.1 Hz) the visual system is predominant, at medium frequencies, vestibular and visual systems interact together to stabilize the gaze while at high frequencies (from 1 to 5 Hz), only the vestibular system enters into action.

When the head rotates rapidly in the horizontal or in the anterior or posterior planes, the two semi-circular channels of each pair participate in the estimation on how fast is the head movement. The ipsilateral canal at rotation gives the main excitatory information - via the vestibular nerve fibers - to which is added the weaker and inhibitory information from the contra-lateral channel. In response, the oculomotor system will induce an eye movement equal in amplitude but in an opposite direction to the movement of the head. This reflex, called vestibulo-ocular reflex (VOR), allows the stabilization of the image on the retina during rapid movements of the head and is estimated during a test known as the functional Head Impulse Test (fHIT) (Halmagyi, GM, and Curthoys, IS (1988). A clinical sign of canal paresis. Arch. Neurol. 45, 737-739).

The VOR is controlled at the central level (Leigh, RG, Zee, DS (2015). *The Neurology of Eye Movements,* 5th edition. Universit Press, Oxford) and specifically the cerebellum has a pivotal role in monitoring and adapting continuously visual gaze. For instance, combining lesion and physiologic studies, the cerebellar regions closely related to ocular motor functions (the VOR, the pursuit and saccadic eye movements). Neurons in the vestibulo-cerebellum (i.e. a region of the cerebellum found in the flocculonodular lobe that receives vestibular and visual information) are activated in relation to head or target motion, during eye fixation and vestibular responses to head motion. Those located in the nodulus and the ventral uvula modulate the velocity-storage mechanism within the vestibular nuclei.

Since several years, the cerebellum is considered as a major role in the cause of most of neurodevelopmental disorders. Part of symptoms such as social communication deficits, executive dysfunctions, poor motor control or memory impairments which are reported in autism spectrum disorders (ASD), dyslexia, Attention Deficit / Hyperactive Disorders (ADHD) may be related to functional deficits in distinct cerebellar sub-regions.

In the present study our goal was to further explore part of the cerebellar functions, specifically those involved in the VOR, also in the specific context of children with neurodevelopmental disorders.

The functional Head Impulse Test (fHIT) has been developed to estimate the VOR during passive head impulses. Halmagyi et Curthoys disclosed in 1988 the "head impulse test", which allows to identify the complete loss of the peripheral vestibular function (see, as a general reference, Halmagyi GM, Curthoys IS. A clinical sign of canal paresis. Arch Neurol. 1988; 45: 737-739).

The rotation of the head may achieve angular accelerations of thousands of degrees per square second.

In other words, the VOR is tested by asking to the subject to identify an optotype briefly presented during active or passive head impulse (Corallo G, Versino M, Mandalà M, Colnaghi S, Ramat S. The functional head impulse test: preliminary data. J Neurol. 2018 Oct; 265 (Suppl 1):35-39. Versino M, Colnaghi S, Corallo G, Mandalà M, Ramat S. The functional head impulse test: Comparing gain and percentage of correct answers. Prog Brain Res. 2019; 248:241-248)

Nowadays, according to the applicant's knowledge, it is not known whether the VOR can be correlated with dyslexia.

The "head impulse test" is based on said second law of Ewald and allows to identify a pathological state even when adapting processes have occurred. While it has been conceived in order to investigate the functionality of the horizontal semicircular canals, it has then been applied to the other semicircular canal pairs as well.

Dyslexia rehabilitation today is carried out without the aid of objective parameters on eye movement but is based on repetitions of words and reading exercises.

### Summary of the invention

The problem addressed by the present invention is therefore that of providing a device that can be effectively employed in the treatment of dyslexia. In particular, said device should be used both in the dyslexia prevention phase and in the dyslexia treatment phase, using a data coming from said device which serves to set up the prevention or treatment of dyslexia.

Furthermore, an additional problem is the identification of a non-therapeutic method of preventing the onset of dyslexia or to improve reading skills or a method that, applied in advance on the subject who may potentially develop dyslexia, avoids the onset of said disease.

Finally, a further problem is the development of a diagnostic method which allows to identify patients potentially suitable for developing dyslexia.

It has now been developed an apparatus and a method which allow to treat dyslexia, both in the preventive and / or rehabilitative phase; both a non-therapeutic method of preventing the onset of dyslexia or to improve reading skills and a diagnostic method to identify potentially dyslexic patients, as defined in the appended claims, whose definitions are integral part of the present description, have also been identified.

The present non-therapeutic method of preventing dyslexia has an improved efficacy with respect to the methods known in the art, since it allows to achieve a very high percentage of success.

Further characteristics and advantages of the medical use of the invention apparatus and of the diagnostic and prevention methods of dyslexia will result from the description of examples of realization of the invention, provided as an indication of the invention.

### Brief description of the figures

FIGURE. 1 shows an embodiment of the invention applied to the treatment of dyslexia.
FIGURE. 2 shows the embodiment of the invention and highlights the (angular) movement of the head.

### Detailed description of the invention

In the present invention and unless otherwise provided, the following definitions are considered.

"Angular acceleration" is defined as the rate of change in angular velocity over time and it may be expressed as degrees or radians per squared second (° / s² or rad / s²). "Passive movements" relate to the movements imposed on the patient's head by the clinician, for instance, bringing the patient's head between hands posed on the cranial-parietal bones and turning the head to some extents to the left or to the right around its vertical axis. Accordingly, the patient cannot predict direction, speed nor the angle of the rotation determined by said motion.

"Active rotation" refers to the movement made actively by the patients themselves by rotating the head.

"Passive rotation" refers to the movement made passively by a person to the patient themselves by rotating the head.

"Loss" is to be intended as the complete loss of the sensitivity of one SCC, which leads to the patient being completely deprived of the vestibular rotational information and thus to the inability to properly stabilize gaze in space and to the impairment of balance.

"Hypofunctionality or deficit", instead, relates to any little or severe damage to the gaze stabilization and balance systems caused by deficit in the vestibular apparatus. Damages may occur because of infections, inflammatory events, pathologies, pharmacologic treatment side-effect, accidents or even traumas.

The "optotypic line" refers to an array of letters on an optotypic chart used for assessing the visual acuity of a patient, such as the Snellen chart, the E Chart, the Landolt C chart or the Golovin-Sivtsev chart (for a general reference, see, for instance, Ricci F. Cerulli L. : "Standardizzazione dei criteri di valutazione dell'acutezza visiva" . Atti I; Congresso Società Italiana Medicina Legale Oculisticas, pag 35-51 Mattioli Ed. 1994).

"Visual target" refers to any images, such as drawings, symbols, numbers or letters of different sizes, which the patient is required to recognize and read.

A "Sloan's letter" refers to a visual target selected among the capital letters S, O, C, D, K, V, R, H, N or Z.

"Rotational angle" is defined as the angle lying on the traverse plane and comprised between the position of a fixed point of the patient's head at the starting position and the position of the same point achieved after the rotation has been imposed. Said angle may be expressed in degrees or in radians. When rotational movements are manually produced, a certain degree of error may occur, such as, for instance ±5° with respect to the rotational angle declared.

An object of the present invention is an apparatus for the treatment of dyslexia comprising:
- a displaying unit (1);
- a head rotational acceleration measuring unit (2); and
- a recording unit (3).

It has in fact been surprisingly found that this device can be effectively used to prevent the onset of dyslexia or in the treatment of dyslexia, or to improve reading skills.

The displaying unit (1), showing the letter to be recognised by the patient, is placed at a suitable distance, for instance between 30 and 350 cm, or about 150 centimeters, in front of the patient, according to standard clinical procedures .

The displaying unit (1) may be any means suitable for showing a visual target for a period of time in the order of few milliseconds. For instance, it can be a screen on which the visual targets are displayed or projected, or it may be a computer monitor.

In a preferred non limiting example, a computer software may govern the selection of the visual target to be displayed, which will be among those above disclosed.

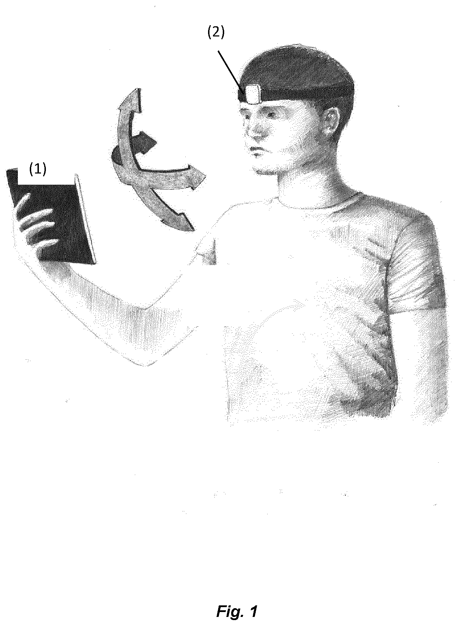

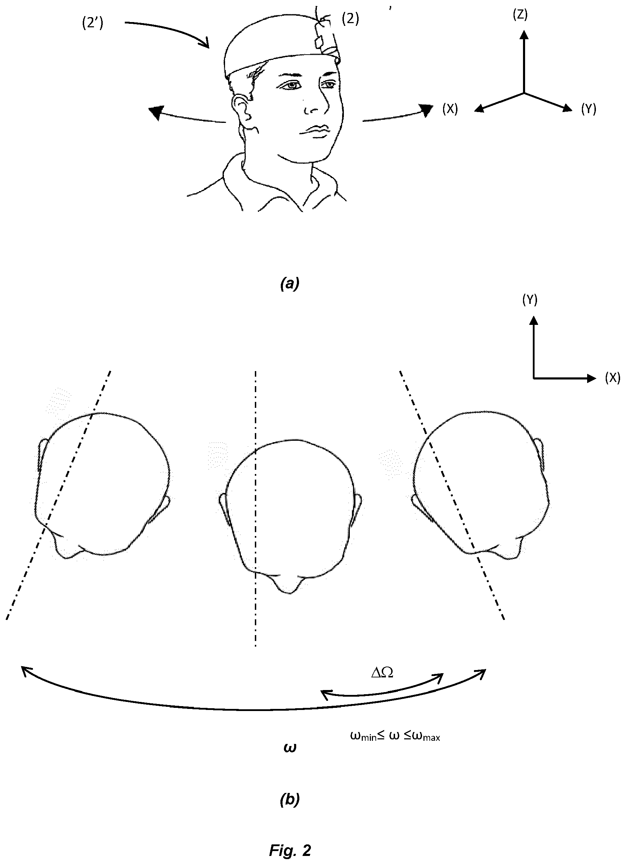

The rotational acceleration measuring unit (2) is any device capable of measuring the angular acceleration, in particular, determined by the rotation imparted to the patient's head by the clinician. Accordingly, a gyroscope and/or an accelerometer may be used, which provide the advantage of being easy to handle. The rotational acceleration measuring unit (2) shall be placed in such a way that it rotates contextually (integrally) with the patient's head, so that they move together. For instance, a helmet or a band may be provided wherein the gyroscope (see, for instance, figure 1 or 2) and/or the accelerometer are fixed thereto. In the case of the two accelerometers, they need to be placed on opposite sides of the axis of the rotation (e.g. one on the forehead and the other one on the nape to measure angular accelerations in the horizontal plane). The measured value of angular acceleration may be acquired by the device itself or, in an alternative embodiment, said unit (2) may be connected to a recording unit (3) directly, which is capable of recording and storing the rotational acceleration data introduced or directly acquired form the unit (2).

The head rotational acceleration measuring unit (2) placed on the patient's head has been equipped with a mode of communication to the other parts of the device that provides for a calculation to prepare the correct test and examination. It calculates the correct head movement in terms of acceleration peak and velocity peak providing the display unit with information on velocity and transmission time.

The device intended as a set of components displaying unit (1) (Reader), head rotational acceleration measuring unit (2) (Sensor) and a recording unit (3) is equipped with an algorithm such as to allow that the display time on the displaying unit is always correct for a specific exam, regardless of the variables and the transmission times that may vary. The times may vary in relation to variables such as useful times for the displaying unit or different processing times. The device for the diagnosis of dyslexia and ADHD recalibrates the display time data in order to allow it to be viewed by a healthy subject more times than by a pathological subject. By detecting the normality detected in scientific researches that used the device in question, it has been found that a certain time resulted in a positive response to the visualization equal to 85% by healthy people and reports this velocity on the diagnosis and screening unit by ensuring that it is correctly calibrated with respect to the main detection unit itself.

The head rotational acceleration measuring unit (2) may be an optical reader or a very high-speed camera which detects the movement of the head.

The head rotational acceleration measuring unit (2) may be a head movement optical reader device which calculates its angular velocity, even in the absence of a sensor (gyroscope and / or accelerometer), but by the simple computerized visual analysis of the movement of the head.

The device calculates and modulates the display time considering that the diagnosis of dyslexia or ADHD has an average response and an average letter displaying time which has been estimated to be approximately 85% (but may vary with further research and data).

In a preferred embodiment, the recording unit (3) may be a computer software.

The recording unit (3) is also able to record the response of the patient. In fact, once the patient has read and recognised the letter shown, they will communicate it to the clinician, who will introduce said result into the recording unit (3). Alternatively, the patient themselves may introduce the response into the unit (2), for instance, with the use of a keyboard in the preferred embodiment, wherein the recording unit (3) is a computer software.

Optionally, the apparatus of the invention may further include an analysing unit (4) . Said analysing unit is connected to the recording unit (3), so that it may acquire the data on patient's responses to be analysed, and may be connected to the measuring unit (2) or to the recording unit (3) in order to acquire data on the rotational acceleration value achieved.

Preferably said analysing unit (4) is connected to the recording unit (3) and to the head rotational acceleration measuring unit (2).

In a preferred embodiment, the recording unit (3) and the analysing unit (4) may be represented by or consisting of a computer software.

The analysing unit (4) is also connected to the displaying unit (1), so that it receives information on the letter shown and compares it to the response given by the patient. In particular, when they do correspond, the patient's response will be correct or positive, otherwise it will be wrong or null. The analysing unit (4) is able to analyse the results collected about rotational acceleration and patient's response in each set of experiments and to provide an output, which may be in any suitable forms, such as, signals, tables or graphs, like column graph, wherein the x and y axes report, for instance, the value of rotational acceleration and the frequency of positive results, respectively.

Accordingly, the trend in frequency of positive results when rotational acceleration increases would thus become evident. In a still further embodiment, the analysing unit (4) may provide an output in the form of signals, visual signal, for instance through the displaying unit (1) or a sound signal, through any suitable means connected thereto, informing, for example, the clinician about the correctness of each response, by comparing the letter shown on the displaying unit (1) and the response of the patient.

According to a preferred embodiment, the head rotational acceleration measuring unit (2) shall be placed in such a way that it rotates contextually (integrally) with the patient's head.

According to a preferred embodiment, the head rotational acceleration measuring unit (2) may be constituted by a gyroscope and / or an accelerometer, or it may be an optical reader or a very high-speed camera which detects the movement of the head.

According to a preferred embodiment, the apparatus further includes an analysing unit (4).

According to a preferred embodiment, the displaying unit (1) may be a screen on which the visual targets are displayed or projected.

According to a preferred embodiment, the displaying unit (1) may be a computer monitor.

According to a preferred embodiment, the recording unit (3) may be a computer software.

According to a preferred embodiment, the recording unit (3) records data on the rotational acceleration value and the response of the patient.

According to a preferred embodiment, the patient themselves introduces the response into the recording unit (3),

According to a preferred embodiment, the recording unit (3) is connected to the rotational acceleration measuring unit (2) and / or to the analysing unit (4).

According to a preferred embodiment, the analysing unit (4) analyses the results collected about rotational acceleration and patient's response.

According to a preferred embodiment, the analysing unit (4) is connected to the displaying unit (1).

According to a preferred embodiment, the analysing unit (4) provides an output.

According to a preferred embodiment, said output may be in form of signals, tables or graphs.

According to a preferred embodiment, the treatment of dyslexia, performed with said apparatus may be used both in the preventive phase, i.e. before the dyslexia disease arises, and / or in the rehabilitation phase, i.e. to treat the patient afflicted with dyslexia (dyslexic).

According to a preferred embodiment, the analysis of the functional level of the vestibulo-ocular reflex (VOR) is integrated in the device, in particular, in the analysis unit (4) to allow the development of prevention and / or of the rehabilitation of dyslexia.

According to a preferred embodiment, the functional level of the VOR of a subject has a deficit calculated by the aforementioned device at the moment of the investigation of the active or passive impulsive head movement, ranging between 100 degrees/s² and 20,000 degrees/s² of peak acceleration of the head movement, said movement ranging between 2 degrees and 45 degrees, preferably about 10 degrees on average.

According to a preferred embodiment, the functional level of the VOR of a subject has a deficit calculated by the aforementioned device at the time of the investigation of the active or passive impulsive head movement, ranging between 10 degrees / s and 800 degrees / sec of peak velocity of the head movement, said movement ranging between 2 degrees and 45 degrees, preferably about 10 degrees on average.

According to a preferred embodiment, in the rehabilitation phase the device, in particular the displaying unit (1), enables the display of a series of words comprising from 2 to 20 letters calculated by an algorithm, wherein said algorithm enables the 2 or 3 letters that have been found to be the most difficult to be read by the subject to be repeated more frequently within the words, during the active or passive impulsive head movement, as defined in the previous two paragraphs.

According to a preferred embodiment, the VOR functional level measurement is carried out by displaying in the displaying unit (1) optokinetic confounding backgrounds and / or confounding videos (to increase the reading difficulty), in the preventive and / or rehabilitation phase of dyslexia.

In other words, preferably, the displaying unit (1) is suitable for displying optokinetic confounding backgrounds and / or confounding videos (to increase reading difficulty), in the preventive and / or rehabilitative phase of dyslexia, to perform the VOR functional level measurement.

Another object is the use of the device described above, including any preferred embodiment, in order to prevent the onset of dyslexia or for the treatment of dyslexia or to improve the reading skills, in particular to decrease errors and increase reading speed.

It has in fact been found that the invention device allows to improve the reading skill, in particular to decrease errors and increase reading speed, both of healthy subjects and subjects suffering from dyslexia. Furthermore, it has been found that the invention device allows to improve the reading skill, both of adults and children; moreover, the invention device allows to improve the reading skill, both of healthy adults and children or of those suffering from dyslexia.

Another object is a non-therapeutic method of preventing the onset of dyslexia or to decrease errors and increase reading speed comprising the following step:
a. using the device described above, to display to the subject, using the displaying unit (1), symbols and / or images during the active or passive impulsive head movement, ranging between 100 degrees/s² and 20,000 degrees/s² of peak acceleration of the head movement and / or ranging from 10 degrees/s to 800 degrees/sec of peak velocity of the head movement, said movement ranging between 2 degrees and 45 degrees, preferably about 10 degrees on average, for a time ranging between 2 minutes and 50 minutes a day.
b. Repeat step a) for a period ranging between 7 days and 300 days, progressively decreasing the time of appearance of the images and / or symbols.

In step b) the time of appearance of the images and / or symbols is progressively decreased, ie the reading velocity is progressively increased.

The term progressively means that, when the reading performance is achieved, this level of difficulty or performance is maintained for a time ranging between one and 2 weeks before increasing the level of difficulty.

During step a) the patient is required, while sitting or standing, to read the letters of an optotypic line on suitably rescaled optotype chart projected onto the displaying unit (1) placed in front of them, usually at a suitable distance comprised for instance between 30 and 250 centimeters, preferably 150 centimeters, as shown in Fig. 1.

During step a), the patient is required to read and recognise a letter shown to them, while the clinician rotates their head imparting a certain rotational acceleration (see, for a general reference, "Principle of the head impulse (thrust) test or Halmagyi head thrust test (HHTT)" F. Wuyts, B-ENT, 2008, 4, Suppl. 8, 23-25). In particular, with reference to Fig. 1, the patient sits or stands in front of a displaying unit (1) at a distance of between about 30 centimetres and about 150 centimeters, preferably being about 50 centimeters, wearing a helmet or a band which measures the velocity and acceleration of the head because of the acceleration measuring unit (2) which is attached to said helmet or band, so that said unit (2) moves contextually (integrally) with the patient's head.

During the execution of the test the clinician stands, for instance, behind the patient and poses his hands on the cranial-parietal bones. In each experiment the clinician rotates the patient's head to the left or to the right around its vertical axis, thus imposing a certain rotational acceleration. Said acceleration value is measured by the measuring unit (2) and may be recorded by the recording unit (3). When said acceleration reaches or overcomes a threshold value formerly set by the clinician, a Sloan's letter appears on the displaying unit (1). Said threshold value may for instance determine a 600 degrees/s² range of acceptable head acceleration; for example, it can be set to about 1000 deg/s², thereby defining a bin ranging between about 700 and about 1300 deg/s² in the course of the first set of experiments, while during the subsequent set the head acceleration may achieve about 1.700-2.300 deg/s².

In particular, with respect to the size of the Sloan's letter shown in said first experiments, this has the size of the letters of the optotypic line immediately superior to the ones read by the patient during the static acuity assessment phase of step a). For instance, if the patient was able to read the letters of the ninth optotypic line, then during the dynamic test the letters belonging to the eight line, having a larger size, are shown. As for the appearance time of the letter, it may be within 10-150 milliseconds and, preferably, between 25-95 milliseconds from the time when the acceleration of the head reaches the threshold. The patient is required to read and to recognise it and the response is then recorded in the recording unit (3).

According to a preferred embodiment, in the step a) the patient is required, while sitting or standing, to read the letters of an optotypic line on an optotype table.

According to a preferred embodiment, in the step a) said optotype table is placed in front of them at a distance comprised between about 30 and about 250 centimeters and preferably 150 centimeters.

According to a preferred embodiment, the step b) includes at least one set of experiments.

According to a preferred embodiment, in each experiment the person is required to read and recognize a letter shown to them, while the clinician rotates their head to the left or to the right around its vertical axis, imparting a certain rotational acceleration.

According to a preferred embodiment, in the step b) the rotational acceleration measuring unit (2) rotates integrally with the patient's head.

According to a preferred embodiment, in the step b) when said acceleration reaches or overcomes a threshold value, a Sloan's letter appears on the displaying unit (1).

According to a preferred embodiment, in the step b) said threshold value is set by the clinician thereby defining an acceptable range of angular accelerations.

According to a preferred embodiment, in the step b) said threshold value may be ranging between about 700 and about 1300 deg / s² in the course of the first set of experiments.

According to a preferred embodiment, in the step b) the Sloan's letter showed in step b) has the size of the letters of the optotypic line immediately superior to the ones read by the patient during the static acuity assessment phase of step a).

According to a preferred embodiment, in the step b) the appearance time of the letter is within 10-120 milliseconds from the time when the acceleration of the head reaches the threshold.

According to a preferred embodiment, each set of experiments of step b) is subsequently repeated for a number of times sufficient to collect a statistically significant number of results with respect to each rotational acceleration.

According to a preferred embodiment, in each repeated set of experiments the rotational angular acceleration is increased of about 1000 degrees / s² with respect to the previous set and up to a value of about 7000 degrees / s².

Another object is a diagnostic method for identifying individuals potentially suitable for developing dyslexia comprising the following steps:
a. to place the person to be diagnosed in front of the device described above and to display, through the displaying unit (1), moving letters;
b. by means of an operator, to subject the person's head to passive movements in the direction of the semicircular canals, where said movements are unpredictable and have an acceleration ranging from 300 to 18,000 degrees / s² of acceleration peak on a head movement between 5 and 20 degrees;
c. using the displaying unit (1) to display the letter or optotype or combination of letters or words for a period of time ranging between 20 milliseconds and 700 milliseconds and to verify the correctness of the reading through a response from the person;
d. using the recording unit (3), to analyze the velocity and accelerations at which the person was unable to make the correct readings;
e. where the person's reading is correct below 85% of the positive responses of a single optotype at an acceleration between 300 and 7000 degrees / s² of maximum acceleration for an appearance time between 30 and 120 milliseconds, that person is potentially capable of developing dyslexia.

It has in fact been found that an incorrect reading under 85% (and 47% on average) of positive responses of a single optotype at a velocity ranging between 300 and 7000 degrees / s² of maximum acceleration (the maximum velocity is equal to acceleration divided by 19.2) for an appearance time between 30 and 120 milliseconds is closely correlated with the dyslexia pathology. Non-dyslexic child on average responds correctly to 94.7%.

According to a preferred embodiment, step a) is performed according to the method just described above, including the preferred embodiments.

According to a preferred embodiment, in step b) in each experiment of each set of experiments the person is required to actively rotate the head from left to right.

According to a preferred embodiment, when the rotational acceleration imparted by said active movements reaches a threshold value, a visual target appears on the displaying unit (1).

According to a preferred embodiment, said threshold value is set by the clinician or by the patient themselves according to the instructions of the clinician in the analysing unit (4).

According to a preferred embodiment, in step b) each experiment is repeated for a number of times decided by the clinician based on their experience.

### EXPERIMENTAL PART

### Materials and Methods

### Subjects

Four different groups of sex-, IQ- and age-matched children participated to the study (Table 1): Group 1 included twenty children with reading impairments, Group 2 enrolled twenty children with ADHD, Group 3 twenty-one children with Autism Spectrum Disorder (ASD) but without intellectual deficiency (ID) and Group 4 was with twenty children with typical neurodevelopment (TD). Subjects from the Groups 1, 2 and 3 were enrolled in the study at the Child and Adolescent Psychiatry Department, Robert Debre Hospital (Paris, France). To be included they should have a neurological exam in the normal range and should be naïve of psychotropic treatment. Children with reading impairment were recruited from the Centre for Language and Learning Disorders, to which they had been referred for a complete evaluation of their difficulties, including an extensive examination of their phonological capabilities. For each child, the time required to read a text passage, text comprehension and the ability to read words and pseudo-words using the L2MA battery (oral Language, written Language, Memory, Attention, Chevrie-Muller et al., 1997) were measured. The diagnosis of ADHD was done according to DSM-5 criteria (APA, 2013) and carried out using the Kiddie-SADS semi-structured interview (Kiddie Schedule for Affective Disorders and Schizophrenia, Goldman et al. 1998). ADHD symptom severity was assessed by using the ADHD Rating Scale-parental report (ADHD-RS).

This scale is based on a large collection of normative data and has demonstrated reliability and discriminant validity in children and adolescents (Du Paul et al. 1998; Collett et al. 2003). Children with ASD were evaluated by the Expert Centre for ASD without ID was based upon evaluation data from the ADI-R (Autism Diagnostic Interview-Revised, by Lord et al. 1994), the ADOS (Autism Diagnostic Observation Schedule, by Lord et al. 2000) and expert clinical judgment based on DSM-5 criteria. To avoid a confounding effect of comorbidity on the main diagnosis, subjects with a comorbid diagnosis of ASD or ADHD were excluded from group 1, subjects with a comorbid diagnosis of ASD or reading impairment were excluded from group 2 and subjects with a comorbid diagnosis of ADHD or reading impairment were excluded from group 3. The mean intelligence quotient (IQ) was evaluated using the Wechsler Intelligence Scale for Children, fifth edition, for all subjects enrolled in groups 1-3. For all them, the IQ total score was in the normal range, i.e., between 85 and 115. The IQ of children with typically development was estimated by using two subtests of the Weschler scale, one assessing his/her verbal ability (the similarities test) and one assessing his/her performance ability (matrix reasoning test). The clinical characteristics of all four groups of children are summarized in Table 1.

The investigation adhered to the principles of the Declaration of Helsinki and was approved by our Institutional Human Experimentation Committee. Written informed consent was obtained from children and their parents after the nature of the procedure was explained.

### Functional head impulse test (fHIT)

This test is based on the ability of a subject to read an optotype briefly presented during impulsive head rotations at varying angular accelerations (Boehler et la. 2010). Several studies have been conducted already by using this test in adults from the general population (Ramat et al. 2012) (Romano et al. 2019). Or with vestibular neuritis (Corallo et al. 2018; Versino et al. 2019; van Dooren et al. 2019).

Briefly, the testing procedure was as follows: the child is seated on a chair placed at 150 cm distance from a computer screen connected with the fHIT device (Figure 1). First, the static visual acuity is assessed using distance-scaled white Landolt C optotypes displayed on screen. All children had a normal visual acuity of 0.2 logMAR. As a consequence, this size was used in the experiment. During the test, child had a head mounted gyroscope to measure head angular velocities. A trained operator performed subsequent head impulses, consisting of brief, small rotatory movements executed with both hands on the head of the child in the plane of each semi-circular canals pair and, at the same time, it was asked to the child to recognize the Landolt C optotype that was been presented on a black PC screen for 80 ms in eight possible orientations (Figure 1A). The child had to recognize the Landolt C orientation and to report it on a keypad showing all possible ring eight orientations (Figure 1B). The operator performed a minimum of 10 head impulses in each direction for each semi-circular canal plane (the left and right horizontal, left anterior and right posterior, right anterior and left posterior directions).

### Data Analysis

Data was divided in 1000 °/s² wide bins based on peak head angular acceleration reached during each head impulse that triggered the display of the Landolt C. Thresholds ranged 2000-7000 deg/s² and the data was therefore organized in six bins for each subject. The fHIT software automatically separated the trials (i.e., the head impulses) according to the acceleration bins defined above and the semi-circular canal stimulated. As established by the fHIT procedure, the performance of a subject was assessed estimating the proportion of appropriated answers (that is when the child recognized the correct orientation of the Landolt C, see Ramat et al. 2012).

### Statistical analysis

Statistical analysis was performed with the Statistica software using the GLM (Advanced Linear Models) with the four groups of children as inter-subject factors, and the percentage of the correct responses for the distinct semi-circular canals stimulated in the two directions (left and right) as within-subject factors. In case of significant effects, we conducted post-hoc comparisons by using Bonferroni correction for multiple comparisons. The effect of a factor was considered significant when the corrected p-value was below 0.05.

### Results

We first explored the percentage of corrected answers in left- and rightward direction for each vestibular canal (horizontal, posterior and anterior, respectively) for each group of children tested (figure 2). We observed a significant group effect (F (3,79) = 47.34, p<0.0001), which remained consistent after Bonferroni post hoc test. The performance of the fHIT of children with TD was significantly better than those of the three groups of children with NDD (all p<0.0001), suggesting that fHIT could differentiate well all these three children pathologies with respect to TD children.

We also estimated the effect of the type of vestibular canal tested (F (2,158) = 21.86, p <0.0001) on the performance. Bonferroni post hoc analysis revealed that the percentage of appropriated responses of the lateral vestibular canal was higher than the percentage of responses reported after posterior and anterior stimulations of the vestibular canals (both, corrected p <0.01), suggesting that lateral vestibular canal is most used that posterior and anterior vestibular canals.

Finally, the ANOVA exhibited two significant interactions (vestibular canal x direction, F (2,158) = 4.41, p <0.01 and vestibular canal x group, F (6,158) = 2.21, p <0.04, respectively). The Bonferroni post hoc analysis showed that the number of appropriated responses for both directions (left and right) of the lateral vestibular canal was significantly higher with respect to the two directions of the posterior and anterior canals (all, corrected p <0.01). The performances measured for the posterior and anterior canals in the children with NDD were significantly lower than those recorded in the lateral canal (both, corrected p <0.001).

### Discussion

The aim of the present study was to explore the VOR by using the fHIT in children. We compared the performance at this test to estimate a potential dysfunctional defect of a loop involving the vestibular, the ocular system and the cerebellum in children with NDD compared to children with TD. Our study showed, for the first time, that this kind of test could be easily conducted in children with TD but also with NDD. We also observed that children with reading impairments, ADHD and ASD, had significantly poor performance on the fHIT.

This finding is in line with previous studies suggesting deficiencies in vestibular capabilities in children with neurodevelopmental disorders that were performed in distinct studies in which one pathology only (dyslexia, ADHD or ASD) has been examined. Indeed, in a recent meta-analyze Van Hecke and colleagues (2019) reported research in which vestibular function has been investigated in children with intellectual developmental disability (IDD), autism spectrum disorder (ASD), attention deficit/ hyperactivity disorder (ADHD) and specific learning disorder (dyslexia). These authors reviewed twenty studies in which central and/or peripheral vestibular deficiencies has been showed in these types of children that could explain the deficits in motor coordination or balance problems observed in this population (Levinson, 1990; O'Reilly et al. 2011; Isaac et al., 2017). Interestingly, Van Hecke and colleagues pointed out the importance to develop a complete vestibular test battery to further characterize the vestibular capability in children with neurodevelopmental disorders because the majority of the studies reported in this meta-analysis were mainly based on evaluation of the horizontal semicircular canals alone; these authors also encouraged clinicians to further explore vestibular functions in these kinds of children because they could be useful for better define the phenotype or behavioral features of children with neurodevelopmental disorders.

The novelty of the present study is that for the first time a simple, non-invasive test for exploring vestibular function of the three vestibular canals has been conducted at the same time in different groups of children with neurodevelopmental disorders (dyslexic, ADHD and ASD) and in a group of TD children. Our finding showed that the fHIT can differentiate well all these three children pathologies with respect to TD children.

As described in the Introduction, the fHIT gives information of the VOR activity and the present findings showed poor capability of the three semicircular canals to compensate the head movements in children with neurodevelopmental disorders in line with the hypothesis of a deficient vestibular activity, that is strictly linked to cerebellum (Meng et al. 2014), already suggested in these kinds of children pathologies.

Taken together all these findings underlined the importance of introducing systematically the vestibular abilities assessment in children with neurodevelopmental disorders in order to improve the diagnosis for these of patients.

### Conclusion

Our findings showed that children with neurodevelopmental disorders had poor performance in the functional head impulse test with respect to typically developing children, suggesting the presence of vestibular dysfunction in children with dyslexia, with attention deficit/ hyperactivity disorder (ADHD) and with autism spectrum disorder (ASD). Vestibular capabilities in children with neurodevelopmental disorders could be used for better refine their diagnosis. The benefits of vestibular rehabilitation in this kind of patients are therefore evident.

It is important to underline that:
- The vestibulo-ocular reflex (VOR) which is specifically involved in the stabilization of the image on the retina during rapid movements of the head, may be explored by using the functional Head Impulse Test (fHIT).
- The number of appropriate responses during the fHIT was significantly lower in children with neurodevelopmental disorders (NDD) than in children with neurotypical development.
- The VOR abnormalities we observed in children with NDD brought further evidence of the strong ties between a loop involving the vestibular system, the ocular system and the cerebellum, and atypical brain development.

The aim of the present study was to test the cerebellum's ability to compensate head movements by testing functional vestibulo-ocular reflex (VOR) responses in children with neurodevelopmental disorders (dyslexia, attention deficit/ hyperactivity disorder (ADHD) and autism spectrum disorder (ASD)) and in a group of typically developmental (TD) children. The functional head impulse test (fHIT) was used to measure functional performance of the VOR during passive head impulses by asking the child to identify an optotype briefly presented during passive head impulse. We measured the percentage of correct responses for each of the three different semicircular canals stimulated in the two (left and right) directions.

Results showed that number of correct responses to the fHIT of the three semi-circular canals in the two directions obtained by the three groups of children with neurodevelopmental disorders were significantly lower to those reported by the group of TD children. In contrast, dyslexic, ADHD and ASD did not differ each other's.

This finding indicated the presence of altered efficiency of vestibulo-ocular reflex in children with dyslexia, ADHD and ASD, in line with the hypothesis of cerebellar deficiencies in these kinds of children pathologies.

### Table and figure legends

Table 1: Clinical characteristics (mean, standard deviation of the test's score) in four groups of children tested (DYS, children with dyslexia; ADHD, children with attention deficit hyperactivity disorder; ASD, children with autism spectrum disorders; TD, children with typical development). For the L2MA test done in children with dyslexia the standard deviation from normal mean is reported.

Figure 1: Experimental set up of the functional Head Impulse Test (fHIT). A: The person is positioned at 0.5 m from a black screen. A gyroscope is tightly strapped on the head by a head band. The person is instructed to fixate a fixation target (not shown) presented on center of the screen. A trained operator delivers randomly directed head impulses in the lateral plane. Eighty milli-seconds after head velocity exceeds 10°/s, a white Landolt C optotype appears for 80 ms. B: After each impulse, eight possible orientations of the Landolt C optotype are shown on the screen, and the person is asked to choose the correct one and to report it on a keypad showing all possible ring eight orientations.

Table 1: Percentage of corrected answer of the Landolt C optotypes in left- and rightward direction for each vestibular canal (A: horizontal, B: posterior and C anterior) for each group of children tested.

**Table 1**

| | ***Group 1*** | ***Group 2*** | ***Group 3*** | ***Group 4*** |
|---|---|---|---|---|
| | DYS | ADHD | ASD | TD |
| | *N* = *20* | N = 20 | *N* = *21* | *N* = *20* |
| **Age (years)** | 9.6 ± 0.2 | 9.5 ± 0.3 | 9.7 ± 0.5 | 9.2 ± 0.4 |
| **ADHD-RS total score** | 5.1 ± 1.8 | 38.7 ± 1.7 | 5.2 ± 1.1 | 4.8 ± 1.2 |

| **L2MA standard deviation from the mean** | | | | |
|---|---|---|---|---|
| *Oral Language* | 2.8 | | | |
| *Written Language* | 2.6 | | | |
| *Memory* | 2.7 | | | |

| **Autism Diagnostic Interview-Revised (ADI-R) scores** | | | | |
|---|---|---|---|---|
| *Social Reciprocal Interaction* | | | 18.5 ± 1.5 | |
| *Communication* | | | 12.5 ± 0.9 | |
| *Stereotyped Patterns of Behaviors* | | | 5.1 ± 0.5 | |

| **Autism Diagnostic Observation Schedule (ADOS) scores** | | | | |
|---|---|---|---|---|
| *Social Reciprocal Interaction* | | | 8.4 ± 0.8 | |
| *Communication* | | | 3.9 ± 0.4 | |

| **Wechsler scale (WISC-IV) scores** | | | | |
|---|---|---|---|---|
| *Verbal Comprehension subscale* | 101 ± 6 | 100 ± 5 | 101 ± 2 | |
| *Perceptual Reasoning subscale* | 99 ± 4 | 98 ± 3 | 97 ± 2 | |
| *Working Memory subscale* | 93 ± 3 | 91 ± 4 | 86 ± 4 | |
| *Processing Speed subscale* | 89 ± 3 | 90 ± 5 | 91 ± 3 | |
| *Similarity test* | 12 ± 1 | 12 ± 2 | 10 ± 2 | 12 ± 1 |
| *Matrix reasoning test* | 11 ± 1 | 10.2 ± 1 | 10.5 ± 1 | 10.8 ± 2 |

The aforementioned experimental tests have shown that the invention device is also effective in the diagnosis and treatment of children suffering from dyslexia or other specific learning disorders (SLD).

In the sample evaluated (6 school-age children) who underwent the visual treatment described (10 sessions of about 5 minutes a day distributed over a month), 3 showed very significant progresses in reading skills, 2 showed significant progresses, 1 showed modest improvements.

## Claims

1. Device for the treatment of dyslexia comprising:
- a displaying unit (1),
- a head rotational acceleration measuring unit (2) and
- a recording unit (3).

2. Device according to claim 1, wherein the rotational acceleration measuring unit (2) is placed in such a way that it rotates simultaneously with the patient's head.

3. Device according to any one of claims from 1 to 2, in which the rotational acceleration measuring unit (2) may be a gyroscope and / or an accelerometer or an optical device for reading the movement of the head which calculates its angular velocity by the computerized visual analysis of the movement of the head.

4. Device according to any one of claims from 1 to 3, further comprising an analysing unit (4).

5. Device according to claim 4, wherein the analysing unit (4) is connected to recording unit (3) and the head rotational acceleration measuring unit (2).

6. Device according to any one of the claims from 4 to 5, in which in the analysing unit (4) is integrated the analysis of the functional level of the vestibulo-ocular reflex (VOR).

7. Device according to claim 6, wherein the functional level of the VOR of a subject has a deficit calculated by the device according to any one of claims 1 to 6 at the moment of the investigation of the active or passive impulsive head movement, ranging between 100 degrees/s² and 20,000 degrees/s² of peak acceleration of the head movement, said movement ranging between 2 degrees and 45 degrees.

8. Device according to any one of claims 6 to 7, wherein the functional level of the VOR of a subject has a deficit calculated by the device according to any one of claims 1 to 5 at the time of the investigation of the active or passive impulsive head movement, ranging between 10 degrees / s and 800 degrees / sec of peak velocity of the head movement, said movement ranging between 2 degrees and 45 degrees.

9. Device according to any one of claims 1 to 8, wherein the displaying unit (1), enables the display of a series of words comprising from 2 to 20 letters calculated by an algorithm, wherein said algorithm enables the 2 or 3 letters that have been found to be the most difficult to be read by the subject to be repeated more frequently within the words, during the active or passive impulsive head movement, as defined according to any one of claims 7 to 8.

10. Device according to any one of claims 1 to 9, wherein the displaying unit (1) is suitable for displaying optokinetic confounding backgrounds and / or confounding videos.

11. Device according to any one of claims 1 to 10, wherein the recording unit (3) and the analysing unit (4) may be a computer software.

12. Use of the device according to any one of claims 1 to 11, in order to prevent the onset of dyslexia or for the treatment of dyslexia or to decrease errors and increase reading speed.

13. Non-therapeutic method of preventing the onset of dyslexia or to decrease errors and increase reading speed comprising the following step:
a. using the device according to any one of claims 1 to 11, to display to the subject, using the displaying unit (1), symbols and / or images during the active or passive impulsive head movement, as defined according to any one of claims 7 to 8, or their combination, for a time ranging between 2 minutes and 50 minutes a day.
b. Repeat step a) for a period ranging between 7 days and 300 days, progressively decreasing the time of appearance of the images and / or symbols.

14. Diagnostic method for identifying individuals potentially suitable for developing dyslexia comprising the following steps:
a. to place the person to be diagnosed in front of the device according to any one of claims 1 to 3 and to display, through the displaying unit (1), moving letters;
b. by means of an operator, to subject the person's head to passive movements in the direction of the semicircular canals, where said movements are unpredictable and have an acceleration ranging from 300 to 18,000 degrees / s² of acceleration peak on a head movement between 5 and 20 degrees;
c. using the displaying unit (1) to display the letter or optotype or combination of letters or words for a period of time ranging between 20 milliseconds and 700 milliseconds and to verify the correctness of the reading through a response from the person; .
d. using the recording unit (3), to analyze the velocity and accelerations at which the person was unable to make the correct readings;
e. where the person's reading is correct below 85% of the positive responses of a single optotype at an acceleration between 300 and 7000 degrees / s² of maximum acceleration for an appearance time between 30 and 120 milliseconds, that person is potentially capable of developing dyslexia.
